(19) **Europäisches Patentamt European Patent Office Office européen des brevets**

(11) **EP 4 786 493 A1**

(12) **EUROPEAN PATENT APPLICATION**
published in accordance with Art. 153(4) EPC

(43) Date of publication:
**05.08.2026 Bulletin 2026/32**

(21) Application number: **24870924.8**

(22) Date of filing: **27.09.2024**

(51) International Patent Classification (IPC):
*C07K 16/28* (2006.01)    *C07K 16/46* (2006.01)
*C07K 19/00* (2006.01)    *C12N 15/13* (2006.01)
*C12N 15/62* (2006.01)    *A61K 39/00* (2006.01)
*A61P 35/00* (2006.01)

(86) International application number:
**PCT/CN2024/121620**

(87) International publication number:
**WO 2025/067386 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311281157**

(71) Applicant: **Nanjing Probio Biotech Co., Ltd.**
**Nanjing, Jiangsu 211100 (CN)**

(72) Inventors:
• **LI, Yingyu**
**Nanjing, Jiangsu 211100 (CN)**

• **XIN, Yang**
**Nanjing, Jiangsu 211100 (CN)**
• **PAN, Qianhui**
**Nanjing, Jiangsu 211100 (CN)**
• **JIA, Wenshuang**
**Nanjing, Jiangsu 211100 (CN)**
• **CHEN, Li**
**Nanjing, Jiangsu 211100 (CN)**

(74) Representative: **Synergy IP Group AG**
**Unterer Rheinweg 50**
**Postfach**
**4001 Basel (CH)**

Remarks:
The complete document including Reference Table(s) and the Sequence Listing(s) can be downloaded from the EPO website

(54) **BINDING PART FOR BINDING CLAUDIN 18.2 AND USE THEREOF**

(57) A binding part for binding with Claudin 18.2 and a use thereof, in particular a fully human antibody that binds with the Claudin 18.2 protein and a use thereof. The antibody specifically binds to Claudin 18.2 without binding to Claudin 18.1, and the provided fully human antibody has high binding activity, low immunogenicity, and high safety.

**EP 4 786 493 A1**

**Description**

**Cross Reference to Related Applications**

The present application claims the right of priority for Chinese patent application no. CN 202311281157.1 filed on September 28, 2023, which is incorporated herein by reference in its entirety.

**Technical Field**

The present invention belongs to the field of tumour immunotherapy and molecular immunology, in particular to the field of antibodies, and specifically relates to a fully human antibody for binding to Claudin 18.2.

**Background Art**

The family of Claudins is extensively found in various epithelial tissues, and different tissues can express different types of Claudin proteins. Claudin 18.2 (tight-junction protein 18.2) is an adhesion molecule that forms tight junctions between cells, and can create a barrier that regulates cell permeability. The human Claudin 18.2 gene is located on chromosome 3 at 3q22.3. Its first exon has two alleles, resulting in two splice variants-Claudin 18.1 and Claudin 18.2. Claudin 18.1 and Claudin 18.2 differ in their N-terminal amino acid sequences and extracellular epitopes, thereby avoiding immunologic cross-reactivity. Currently, Claudin 18.2 is believed to be primarily regulated by the CpG island promoter (a hypomethylated gene sequence within the gene coding sequence of Claudin 18.2), and cAMP-response element binding protein (CREB) (a transcription factor). Claudin 18.1 is mainly expressed in the lung and rarely in other tissues; whereas Claudin 18.2 is expressed in epithelial cells of the gastric mucosa, with particularly high expression in tissues such as gastric cancer, pancreatic cancer, and oesophageal cancer, but rarely in the lung tissue. The high tissue specificity of Claudin 18.1 and Claudin 18.2 forms the molecular basis for Claudin 18.2 as a therapeutic target.

During the process of malignant transformation of cells, cell polarity changes, and Claudin 18.2 becomes exposed on the surface of tumour cells, making it possible to be targeted by antibodies for binding and an ideal target for monoclonal antibody development, especially for solid tumour immunotherapy. Claudin 18.2 can serve as a potential specific marker for tumour diagnosis and treatment. Abnormal activation and overexpression of Claudin 18.2 have been observed in various types of malignancies: approximately 70% of gastric cancer patients may exhibit Claudin 18.2 overexpression in their primary tumours, with rates of 50% and 30% in pancreatic and oesophageal cancers, respectively.

Currently, several immunotherapy strategies against the Claudin 18.2 target have been developed, including monoclonal antibodies (mAbs), bispecific antibodies (BsAbs), chimeric antigen receptor T-cell (CAR-T) therapies, and antibody-drug conjugates (ADCs). Among them, the most advanced drug, Zolbetuximab, is a chimeric mouse monoclonal antibody with a human immunoglobulin 1 constant region. However, technical challenges still remain in the development of Claudin 18.2 antibodies. Firstly, from a protein structural perspective, Claudin 18.2 has 4 transmembrane domains, two extracellular loops, with the $NH_2$ and COOH termini located intracellularly. The extracellular loops of multi-pass transmembrane proteins are not independent domains. Expressing only the extracellular segments cannot maintain the correct conformation; restraint by the transmembrane segments is required to preserve the correct conformation. This significantly increases the difficulty of protein expression. Therefore, finding antigen protein materials that can fully present the two extracellular loops of the protein for subsequent antibody panning is a fundamental step in initiating research. Secondly, both Claudin 18.1 and Claudin 18.2 consist of 261 amino acids with a high structural homology. Claudin 18.1 and Claudin 18.2 differ by only 7 amino acid residues within the approximately 50-amino-acid sequence of the extracellular loop ECL1. Thus, obtaining antibodies for specifically binding to Claudin 18.2 is crucial. As is well known, humanized or fully human antibodies have low immunogenicity and represent the future development direction of antibodies. However, due to technical difficulties, no reported humanized or fully human antibodies for specifically binding to Claudin 18.2 have been approved for marketing. Therefore, there is a need to develop a humanized or fully human antibody for specifically binding to Claudin 18.2.

**Summary**

In a first aspect, the present invention provides a binding part for specifically binding to Claudin 18.2, comprising

a heavy chain variable region (VH), wherein the VH comprises: a VH CDR1, a VH CDR2, and a VH CDR3; wherein the VH CDR1, the VH CDR2, and the VH CDR3 comprise:

(1) amino acid sequences as set forth in SEQ ID NOs: 2, 3, and 4, respectively;
(2) amino acid sequences as set forth in SEQ ID NOs: 10, 11, and 12, respectively; or

(3) amino acid sequences as set forth in SEQ ID NOs: 28, 3, and 29, respectively,

or a variant thereof comprising up to 3 amino acid substitutions in the CDRs; and
(b) a light chain variable region (VL), wherein the VL comprises: a VL CDR1, a VL CDR2, and a VL CDR3; wherein the VL CDR1, the VL CDR2, and the VL CDR3 comprise:

(1) an amino acid sequence as set forth in SEQ ID NO: 6, EVT, and an amino acid sequence as set forth in SEQ ID NO: 8, respectively;
(2) an amino acid sequence as set forth in SEQ ID NO: 14, KVS, and an amino acid sequence as set forth in SEQ ID NO: 16, respectively;
(3) an amino acid sequence as set forth in SEQ ID NO: 19, EVS, and an amino acid sequence as set forth in SEQ ID NO: 21, respectively;
(4) an amino acid sequence as set forth in SEQ ID NO: 24, DTS, and an amino acid sequence as set forth in SEQ ID NO: 26, respectively;
(5) an amino acid sequence as set forth in SEQ ID NO: 31, SNN, and an amino acid sequence as set forth in SEQ ID NO: 33, respectively;
(6) an amino acid sequence as set forth in SEQ ID NO: 36, AAS, and an amino acid sequence as set forth in SEQ ID NO: 38, respectively;
(7) an amino acid sequence as set forth in SEQ ID NO: 40, GAS, and an amino acid sequence as set forth in SEQ ID NO: 42, respectively; or
(8) an amino acid sequence as set forth in SEQ ID NO: 45, WAS, and an amino acid sequence as set forth in SEQ ID NO: 47, respectively,

or a variant thereof comprising up to 3 amino acid substitutions in the CDRs.

In some embodiments,

the VH CDR1, the VH CDR2, and the VH CDR3 comprise the amino acid sequences as set forth in SEQ ID NOs: 2, 3, and 4, respectively, and the VL CDR1, the VL CDR2, and the VL CDR3 comprise the amino acid sequence as set forth in SEQ ID NO: 6, EVT, and the amino acid sequence as set forth in SEQ ID NO: 8, respectively;
the VH CDR1, the VH CDR2, and the VH CDR3 comprise the amino acid sequences as set forth in SEQ ID NOs: 2, 3, and 4, respectively, and the VL CDR1, the VL CDR2, and the VL CDR3 comprise the amino acid sequence as set forth in SEQ ID NO: 19, EVS, and the amino acid sequence as set forth in SEQ ID NO: 21, respectively;
the VH CDR1, the VH CDR2, and the VH CDR3 comprise the amino acid sequences as set forth in SEQ ID NOs: 2, 3, and 4, respectively, and the VL CDR1, the VL CDR2, and the VL CDR3 comprise the amino acid sequence as set forth in SEQ ID NO: 24, DTS, and the amino acid sequence as set forth in SEQ ID NO: 26, respectively;
the VH CDR1, the VH CDR2, and the VH CDR3 comprise the amino acid sequences as set forth in SEQ ID NOs: 2, 3, and 4, respectively, and the VL CDR1, the VL CDR2, and the VL CDR3 comprise the amino acid sequence as set forth in SEQ ID NO: 36, AAS, and the amino acid sequence as set forth in SEQ ID NO: 38, respectively;
the VH CDR1, the VH CDR2, and the VH CDR3 comprise the amino acid sequences as set forth in SEQ ID NOs: 2, 3, and 4, respectively, and the VL CDR1, the VL CDR2, and the VL CDR3 comprise the amino acid sequence as set forth in SEQ ID NO: 40, GAS, and the amino acid sequence as set forth in SEQ ID NO: 42, respectively;
the VH CDR1, the VH CDR2, and the VH CDR3 comprise the amino acid sequences as set forth in SEQ ID NOs: 10, 11, and 12, respectively; and the VL CDR1, the VL CDR2, and the VL CDR3 comprise the amino acid sequence as set forth in SEQ ID NO: 14, KVS, and the amino acid sequence as set forth in SEQ ID NO: 16, respectively;
the VH CDR1, the VH CDR2, and the VH CDR3 comprise the amino acid sequences as set forth in SEQ ID NOs: 28, 3, and 29, respectively; and the VL CDR1, the VL CDR2, and the VL CDR3 comprise the amino acid sequence as set forth in SEQ ID NO: 31, SNN, and the amino acid sequence as set forth in SEQ ID NO: 33, respectively; or
the VH CDR1, the VH CDR2, and the VH CDR3 comprise the amino acid sequences as set forth in SEQ ID NOs: 28, 3, and 29, respectively; and the VL CDR1, the VL CDR2, and the VL CDR3 comprise the amino acid sequence as set forth in SEQ ID NO: 45, WAS, and the amino acid sequence as set forth in SEQ ID NO: 47, respectively.

In some embodiments, the VL is a Kappa light chain variable region or a Lambda light chain variable region.
In some embodiments, the VH comprises an amino acid sequence having at least 80% sequence identity to an amino acid sequence selected from the group consisting of: SEQ ID NOs: 1, 9, 17, 22, 27, 34, and 43; or the VL comprises an amino acid sequence having at least 80% sequence identity to an amino acid sequence selected from the group consisting of: SEQ ID NOs: 5, 13, 18, 23, 30, 35, 39, and 44.
In some embodiments, the VH and VL comprise:

(1) the amino acid sequences as set forth in SEQ ID NOs: 1 and 5, respectively;
(2) the amino acid sequences as set forth in SEQ ID NOs: 9 and 13, respectively;
(3) the amino acid sequences as set forth in SEQ ID NOs: 17 and 18, respectively;
(4) the amino acid sequences as set forth in SEQ ID NOs: 22 and 23, respectively;
(5) the amino acid sequences as set forth in SEQ ID NOs: 27 and 30, respectively;
(6) the amino acid sequences as set forth in SEQ ID NOs: 34 and 35, respectively;
(7) the amino acid sequences as set forth in SEQ ID NOs: 34 and 39, respectively; or
(8) the amino acid sequences as set forth in SEQ ID NOs: 43 and 44, respectively.

In some embodiments, the binding part is sdab, HcAb, Fab, Fab', $F(ab')_2$, Fv, scFv, $(scFv)_2$, or a full-length antibody.

In some embodiments, the binding part is a mouse binding part, a chimeric binding part, a humanized binding part, or a fully human binding part.

In some embodiments, the binding part is a fully human antibody.

In some embodiments, the binding part is a fully human full-length antibody.

In a second aspect, the present invention provides a multispecific antibody or a binding fragment thereof, comprising the binding part of the first aspect.

In a third aspect, the present invention provides a chimeric antigen receptor, comprising:

(a) an extracellular antigen-binding domain, comprising the binding part of the first aspect, or the multispecific antibody or the binding fragment thereof of the second aspect;
(b) a transmembrane domain; and
(c) an intracellular signalling domain.

In a fourth aspect, the present invention provides a nucleic acid encoding the binding part of the first aspect, the multispecific antibody or the binding fragment thereof according to the second aspect, or the chimeric antigen receptor of the third aspect.

In a fifth aspect, the present invention provides a host cell, comprising the nucleic acid of the fourth aspect.

In a sixth aspect, the present invention provides a pharmaceutical composition, comprising the binding part of the first aspect, the multispecific antibody or the binding fragment thereof of the second aspect, the chimeric antigen receptor of the third aspect, or the host cell of the fourth aspect, and a pharmaceutically acceptable carrier.

In a seventh aspect, the present invention provides a method for treating a disease associated with the expression of Claudin 18.2 in a subject in need thereof, comprising administering to the subject a therapeutically effective amount of the pharmaceutical composition of the sixth aspect.

In some embodiments, the disease associated with the expression of Claudin 18.2 is gastric cancer, pancreatic cancer, oesophageal cancer, breast cancer, colon cancer, liver cancer, ovarian cancer, or lung cancer.

In an eighth aspect, the present invention provides a use of the binding part of the first aspect, the multispecific antibody or the binding fragment thereof of the second aspect, the chimeric antigen receptor of the third aspect, the nucleic acid of the fourth aspect, or the host cell of the fifth aspect, in the manufacture of a medicament for treating a disease associated with the expression of Claudin 18.2.

In some embodiments, the disease associated with the expression of Claudin 18.2 is gastric cancer, pancreatic cancer, oesophageal cancer, breast cancer, colon cancer, liver cancer, ovarian cancer, or lung cancer.

The beneficial effects of the present invention:

The amino acid sequences of fully human antibodies are 100% derived from humans, eliminating the need for a subsequent humanization process. Compared to commonly used murine or chimeric antibodies, fully human antibodies have lower rejection reactions, better safety, higher rates of druggability, and represent the development trend for therapeutic antibodies. The 8 fully human antibodies targeting Claudin 18.2 provided by the present invention were generated through multi-step screening using Nanjing ProBio's antibody production platform. Specifically, liquid-phase panning was performed using Claudin 18.2 proteins in a near-native conformation; cell screening was performed using Claudin 18.2-overexpressing cell lines; and Claudin 18.1 was used as a negative control for panning. Compared to transgenic mouse platforms, this antibody production platform is based on screening from fully human native libraries, yielding promising candidate molecules. This antibody production platform offers lower costs (no transgenic mouse licensing fees), shortens the cycle by 1-2 months, significantly accelerates the fully human antibody discovery process, and increases the probability of screening suitable molecules. The antibodies provided by the present invention for binding to Claudin 18.2 exhibit high binding activity towards Claudin 18.2, with $EC_{50}$ values less than 1 nM, even less than 0.1 nM; and affinity (KD) less than 10 nM, which is higher than that of IMAB362. The antibody AHP33029 of the present invention demonstrates superior CDC activity compared to IMAB362.

## Brief Description of the Drawings

FIG. 1 shows the ELISA $EC_{50}$ assay results of fully human antibodies and human Claudin 18.2 proteins.

FIG. 2 shows the FACS $EC_{50}$ assay results of fully human antibodies and HEK293-overexpressing cells expressing human Claudin 18.2 proteins.

FIG. 3 shows the FACS $EC_{50}$ assay results of fully human antibodies and HEK293-overexpressing cells expressing murine Claudin 18.2 proteins.

FIG. 4 shows the FACS $EC_{50}$ assay results of fully human antibodies and HEK293-overexpressing cells expressing human Claudin 18.1 proteins.

FIG. 5 shows the sensorgram for affinity assay of fully human antibodies and human Claudin 18.2 proteins.

FIG. 6 shows the epitope assay results of fully human antibodies based on IMAB362.

FIG. 7 shows the *in vitro* CDC assay results of fully human antibodies targeting human Claudin 18.2.

FIG. 8 shows the *in vitro* ADCC assay results of fully human antibodies targeting human Claudin 18.2.

## Detailed Description of Embodiments

The present invention relates to an antibody for binding to the Claudin 18.2 target and a use thereof. The embodiments of the present invention will be described in detail below in combination with the Examples. Unless otherwise stated, the technical and scientific terms used in the present invention have the same meanings as commonly understood by those of ordinary skill in the art to which the present invention belongs.

As used herein, the term "a/an" refers to one or more (i.e., at least one) grammatical object designated by the article. For example, "an antibody" means one antibody or more than one antibody.

As used herein, the term "binding part" refers to a molecule or a part of a molecule for binding to a target molecule (e.g., Claudin 18.2). The binding part may include a protein, peptide, nucleic acid, carbohydrate, lipid, or small molecular weight compound. In some embodiments, the binding part includes an antibody. In some embodiments, the binding part includes an antigen-binding fragment of an antibody. In some embodiments, the binding part includes a small molecular weight component. The binding part can also be an antibody or an antigen-binding fragment thereof. In some embodiments, the binding part includes a ligand-binding domain of a receptor. In some embodiments, the binding part includes an extracellular domain of a transmembrane receptor. The binding part can also be a ligand-binding domain of a receptor or an extracellular domain of a transmembrane receptor. The binding part may be monovalent, meaning that it contains one binding site that specifically interacts with the target molecule. The binding part may also be bivalent, meaning that it contains two binding sites that specifically interact with the target molecule. The binding part may also be multivalent, meaning that it contains multiple binding sites that specifically interact with the target molecule. The bivalent or multivalent binding parts may interact with one or more epitopes on a single target molecule. The bivalent or multivalent binding parts may also interact with two or more target molecules.

As used herein, the term "binding affinity" generally refers to the strength of the sum of non-covalent interactions between a binding part and a target molecule. The binding of a binding part with a target molecule is a reversible process, and the affinity of binding is typically reported as the equilibrium dissociation constant (KD). The KD is the ratio of the dissociation rate (koff or kd) to the association rate (kon or ka). The lower the KD of a binding pair, the higher the affinity. Various methods for measuring the binding affinity are known in the art, any of which can be used for the purposes of the present disclosure. Specific illustrative embodiments include each of the following. In one embodiment, the "KD" or "KD value" can be measured by assays known in the art, for example, by a binding assay. The KD can be measured by radioimmunoassay (RIA) (Chen et al., (1999) J. Mol Biol 293:865-881). The KD or KD value can also be measured by Biacore surface plasmon resonance assay using, e.g., a BIAcoreTM-2000 or BIAcoreTM-3000 (BIAcore, Inc., Piscataway, NJ), or by biolayer interferometry using, e.g., an OctetQK384 system (ForteBio, Menlo Park, CA). When a target molecule containing multiple epitopes contacts a binding part containing multiple binding sites for binding the target molecule, the interaction of the binding molecule at one site with the target molecule will increase the probability of reaction at a second site. The force of such multiple interactions between a multivalent antibody and an antigen is called avidity. For example, high avidity can compensate for low affinity, as sometimes found with pentameric IgM antibodies, which may have lower affinity than IgG, but the high avidity of IgM due to multivalency allows it to bind with an antigen effectively. In some embodiments, the binding part provided herein has a binding affinity KD value for Claudin 18.2 of less than 10 nM; in some embodiments, the binding part described herein has a binding affinity KD value for Claudin 18.2 of less than 5 nM; and in some embodiments, the binding part described herein has a binding affinity KD value for Claudin 18.2 of less than 1 nM.

As used herein, the terms "target(s)/targeting", "specifically bind(s)/binding", and "bind(s)/binding" mean that a polypeptide or molecule interacts with an epitope, protein, or target molecule more frequently, more rapidly, for a longer duration, with greater affinity, or with a certain combination thereof compared to alternative substances including related and unrelated proteins. A binding part (e.g., antibody) for specifically binding to a target molecule (e.g., antigen) can be identified, for example, by immunoassay, ELISA, SPR (e.g., Biacore), or other techniques known to those skilled in the art. Typically, a specific reaction will be at least twice the background signal or noise, and may be more than 10 times the background signal. For a discussion of antibody specificity, see, e.g., Paul, ed., 1989, Fundamental Immunology Second Edition, Raven Press, New York, pp. 332-336. A binding part for specifically binding to a target molecule binds said target molecule with an affinity that may be higher than its affinity for other different molecules. In some embodiments, a binding part for specifically binding to a target molecule binds said target molecule with an affinity that may be at least 20 times, at least 30 times, at least 40 times, at least 50 times, at least 60 times, at least 70 times, at least 80 times, at least 90 times, or at least 100 times greater than its affinity for other different molecules. In some embodiments, a binding part for specifically binding to a particular target molecule binds other different molecules with such low affinity that the binding is not detectable using assays described herein or known in the art. In some embodiments, "specifically bind(s)/binding" means that, for example, a binding part has a KD for a target molecule of about 0.1 mM or less. In some embodiments, "specifically bind(s)/binding" means that a polypeptide or molecule has a KD for a target molecule of about 10 $\mu$M or less, or about 1 $\mu$M or less. In some embodiments, "specifically bind(s)/binding" means that a polypeptide or molecule has a KD for a target molecule of about 0.1 $\mu$M or less, or about 1 nM or less. Due to the sequence identity among homologous proteins in different species, specific binding can include polypeptides or molecules that recognize a protein or target in more than one species. Similarly, due to the homology within certain regions of polypeptide sequences of different proteins, specific binding can include polypeptides or molecules that recognize more than one protein or target. It should be understood that in some embodiments, a binding part for specifically binding to a first target may or may not specifically bind with a second target. Thus, "specifically bind(s)/binding" does not necessarily require (but can include) exclusive binding, i.e., binding to a single target. Therefore, in some embodiments, a binding part may specifically bind to more than one target. For example, in some cases, an antibody may comprise two identical antigen-binding sites, each of which specifically binds to the same epitope on two or more proteins. In certain alternative embodiments, an antibody may be bispecific and comprises at least two antigen-binding sites with different specificities. In some embodiments, the binding part specifically binds to human Claudin 18.2. In some embodiments, the binding part specifically binds to murine Claudin 18.2. In some embodiments, the binding part specifically binds to cynomolgus monkey Claudin 18.2.

As used herein, the term "binding part" means a part capable of binding to an antigen, including an antibody and a binding fragment thereof. As used herein, the term "antibody" refers to an immunoglobulin molecule that recognizes and specifically binds to a target via at least one antigen-binding site, such as a protein, polypeptide, peptide, carbohydrate, polynucleotide, lipid, or any combination of the foregoing, where the antigen-binding site is typically within the variable region of the immunoglobulin molecule. As used herein, the term encompasses a full-length polyclonal antibody, full-length monoclonal antibody, single-chain Fv (scFv) antibody, light chain antibody (LCAb), multispecific antibody, bispecific antibody, monospecific antibody, monovalent antibody, fusion protein comprising the antigen-binding site of an antibody, and any other modified immunoglobulin molecule comprising an antigen-binding site (e.g., dual variable domain immunoglobulin molecules), as long as the antibody exhibits the desired biological activity. Antibodies also include, but are not limited to, mouse antibodies, chimeric antibodies, humanized antibodies, and human antibodies. Antibodies can be of any of the five major classes of immunoglobulins: IgA, IgD, IgE, IgG, and IgM, or subclasses (isotypes) thereof (e.g., IgG1, IgG2, IgG3, IgG4, IgA1, and IgA2), based on the identity of the heavy chain constant domain, called $\alpha$, $\delta$, $\epsilon$, $\gamma$, and $\mu$, respectively. Different classes of immunoglobulins have different and well-known subunit structures and three-dimensional configurations. Antibodies can be naked or conjugated to other molecules, including, but not limited to, toxins and radioisotopes. Unless explicitly indicated otherwise, the term "antibody" as used herein includes "antigen-binding fragments" of intact antibodies.

In some embodiments, the antibody described herein is a monoclonal antibody. In some embodiments, the antibody described herein is a fully human monoclonal antibody. In some embodiments, the antibody described herein is a fully human full-length antibody.

As used in connection with an antibody, the term "antigen-binding fragment" refers to a part of an intact antibody and refers to an antigen-determining variable region of the intact antibody. Examples of antibody fragments include, but are not limited to, Fab, Fab', F(ab')2, Fv, linear antibodies, single-chain antibody molecules (e.g., scFv), light chain antibodies (LCAb), heavy chain antibodies (HcAb), disulfide-linked scFv (dsscFv), bispecific antibodies, trispecific antibodies, tetraspecific antibodies, minibodies, dual variable domain antibodies (DVD), and multispecific antibodies formed from antibody fragments.

As used herein, the term "variable region" of an antibody refers to a light chain variable region of an antibody or a heavy chain variable region of an antibody, either individually or in combination. Generally, each of the heavy and light chain variable regions consists of four framework regions (FRs) and three complementarity determining regions (CDRs) (also known as "hypervariable regions"). The CDRs within each chain are held together in close proximity by the framework regions, and together with the CDRs from the other chain, contribute to the formation of the antigen-binding site of the antibody. There are at least two techniques for determining CDRs: (1) a method based on cross-species sequence variability (Kabat et al., 1991, Sequences of Proteins of Immunological Interest (5th Edition) Bethesda, MD: National Institutes of Health); and (2) a method based on crystallographic studies of antigen-antibody complexes (Al-Lazikani et al., 1997, J. Mol. Biol., 273(4): 927-48). Furthermore, a combination of these two methods is used in the art and can be used to determine CDRs. In some embodiments, the binding part of the present invention comprises a heavy chain variable region that is an IgG1 heavy chain variable region. In some embodiments, the binding part of the present invention comprises a light chain variable region that is a kappa light chain variable region. In some embodiments, the light chain variable region is a lambda light chain variable region. In some embodiments, the kappa light chain variable region sequence comprises an amino acid sequence as set forth in SEQ ID NO: 13, 35, 39, or 44. In some embodiments, the lambda light chain variable region sequence comprises an amino acid sequence as set forth in SEQ ID NO: 5, 18, 23, or 30.

The term "single-chain variable fragment" or "scFv" refers to a fusion protein of heavy and light chain variable regions of an immunoglobulin linked by a short linker peptide of ten to twenty-five amino acids. The linker is typically glycine-rich for flexibility and serine- or threonine-rich for solubility. The scFv retains the specificity of the original immunoglobulin. scFvs can be linked by linkers of different lengths to form di-scFv, bispecific antibodies, tri-scFv, trispecific antibodies, or tetraspecific antibodies, which can exhibit specificity for one or more antigens.

The term "chimeric antigen receptor" or "CAR" refers to an engineered receptor that usually grafts defined specificity onto an immune effector cell, typically a T cell, and enhances T cell function. Next-generation CARs include an extracellular binding domain comprising an scFv, as well as a hinge region, a transmembrane domain, and an intracellular signalling domain (primarily the CD3-$\zeta$ cytoplasmic domain (which is the primary transmitter of T cell activation signals) plus one or more co-stimulatory domains). The CARs can be further modified by adding factors that enhance T cell expansion, persistence, and anti-tumour activity, such as cytokines and co-stimulatory ligands.

As used herein, the term "chimeric antibody" refers to an antibody in which the amino acid sequences of the immunoglobulin molecules are derived from two or more species. Typically, the light and heavy chain variable regions correspond to the variable regions from an antibody of one mammalian species (e.g., mouse, rat, rabbit) having the desired specificity, affinity, and/or binding ability, while the constant regions are homologous to sequences from an antibody of another species (typically human) to avoid eliciting an immune response in said species *in vivo*.

As used herein, the term "fully human antibody" refers to an antibody prepared using any technique known in the art produced by human cells or having an amino acid sequence corresponding to the antibody produced by a human.

As used interchangeably herein, the terms "polypeptide," "peptide," and "protein" refer to a polymer of amino acids of any length, which may be linear or branched. It may comprise non-natural or modified amino acids, or may be interspersed with non-amino acids. Polypeptides, peptides, or proteins may also be modified by, for example, disulfide bond formation, glycosylation, lipidation, acetylation, phosphorylation, or any other manipulation or modification.

As used interchangeably herein, the terms "polynucleotide" and "nucleic acid" refer to a polymer of nucleotides of any length and include DNA and RNA. The nucleotides may be deoxyribonucleotides, ribonucleotides, modified nucleotides or bases, and/or analogues thereof, or any substrate that can be incorporated into a polymer by DNA or RNA polymerases.

As used herein in the context of two or more nucleic acids or polypeptides, the term "identical" or percentage of "identity" refers to two or more sequences or subsequences that are identical or have a specified percentage of nucleotides or amino acid residues that are the same, when compared and aligned (introducing gaps, if necessary) for maximum identity, not considering any conservative amino acid substitutions as part of the sequence identity. The percentage of identity can be measured using sequence comparison software or algorithms or by visual inspection. Various algorithms and software that can be used to obtain alignments of amino acid or nucleotide sequences are well-known in the art. These include, but are not limited to, BLAST, ALIGN, Megalign, BestFit, the GCG Wisconsin Package, and variations thereof. In some embodiments, two nucleic acids or polypeptides of the present invention are substantially identical, meaning that they have at least 70%, at least 75%, at least 80%, at least 85%, at least 90%, and in some embodiments at least 95%, 96%, 97%, 98%, 99% nucleotide or amino acid residue identity, as measured using sequence comparison algorithms or by visual inspection, when compared and aligned for maximum correspondence. In some embodiments, identity exists over a region of amino acid sequences that is at least about 10 residues, at least about 20 residues, at least about 40 to 60 residues, at least about 60 to 80 residues, or any integer value therebetween in length. In some embodiments, identity exists over a region that is more than 60 to 80 residues in length, e.g., at least about 80 to 100 residues, and in some embodiments, the sequences are substantially identical over the full length of the sequences being compared (e.g., the coding region of a target protein or antibody). In some embodiments, identity exists over a region of nucleotide sequences that is at least about 10 bases, at least about 20 bases, at least about 40 to 60 bases, at least about 60 to 80 bases, or any integer value therebetween in length. In some embodiments, identity exists over a region that is more than 60 to 80 bases in length, e.g., at least about 80 to 1000 bases or more, and in some embodiments, the sequences are substantially identical over the full length of the sequences being compared (e.g., the nucleotide sequence encoding a protein of interest).

As used herein, the term "amino acid substitution" refers to the replacement of one amino acid residue with another amino acid residue in a polypeptide sequence. A "conservative amino acid substitution" is one in which one amino acid residue is replaced with another amino acid residue having a side chain with similar chemical characteristics. Families of amino acid residues having similar side chains have been generally defined in the art, including basic side chains (e.g., lysine, arginine, histidine), acidic side chains (e.g., aspartic acid, glutamic acid), polar uncharged side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), β-branched side chains (e.g., threonine, valine, isoleucine), and aromatic side chains (e.g., tyrosine, phenylalanine, tryptophan, histidine). For example, substitution of tyrosine with phenylalanine is a conservative substitution. Generally, conservative substitutions in the sequences of the polypeptide, soluble protein, and/or antibody of the present disclosure do not eliminate binding of the polypeptide, soluble protein, or antibody containing said amino acid sequence to the target binding site. Methods for identifying conservative amino acid substitutions that do not eliminate the binding are well-known in the art.

As used herein with respect to a binding part (e.g., antibody) ("reference binding part") comprising a polypeptide having a particular sequence feature, the term "variant" refers to a different binding part that, compared to the reference binding part, has a polypeptide comprising one or more (e.g., about 1 to about 25, about 1 to about 20, about 1 to about 15, about 1 to about 10, or about 1 to about 5) amino acid sequence substitutions, deletions, and/or additions. An anti-Claudin 18.2 binding part variant or anti-Claudin 18.2 antibody variant retains at least specific binding to Claudin 18.2. In some embodiments, a binding part variant may result from one or more (e.g., about 1 to about 25, about 1 to about 20, about 1 to about 15, about 1 to about 10, about 1 to about 5, or about 1 to about 3) changes in the amino acid sequence of the reference binding part. In addition, for example, an anti-Claudin 18.2 antibody variant may result from one or more (e.g., about 1 to about 25, about 1 to about 20, about 1 to about 15, about 1 to about 10, about 1 to about 5, or about 1 to about 3) changes in the amino acid sequence of a reference anti-Claudin 18.2 antibody. The changes in the amino acid sequence may be amino acid substitutions. In some embodiments, the changes in the amino acid sequence may be conservative amino acid substitutions. In some embodiments, the anti-Claudin 18.2 binding part variant or anti-Claudin 18.2 antibody variant may result from one or more (e.g., about 1 to about 25, about 1 to about 20, about 1 to about 15, about 1 to about 10, about 1 to about 5, or about 1 to about 3) amino acid substitutions in the VH or VL region or sub-region (e.g., one or more CDRs). In some embodiments, the anti-Claudin 18.2 binding part variant or anti-Claudin 18.2 antibody variant may result from one, up to two, up to three, up to four, or up to five amino acid substitutions in each of the VH or VL region. In some embodiments, the anti-Claudin 18.2 binding part variant or anti-Claudin 18.2 antibody variant may result from one, up to two, up to three, up to four, or up to five amino acid substitutions in each of the CDR regions.

The term "vector" refers to a substance used to carry or contain a nucleic acid sequence (e.g., for introducing the nucleic acid sequence into a host cell). The vectors suitable for use include, for example, expression vectors, plasmids, phage vectors, viral vectors, episomes, and artificial chromosomes, which may include selectable sequences or markers operable for stable integration into the chromosome of a host cell. Additionally, the vectors may comprise one or more selectable marker genes and appropriate expression control sequences. The selectable marker genes that may be included provide, for example, resistance to antibiotics or toxins, complement auxotroph, or supply essential nutrients not present in the medium. The expression control sequences may include constitutive and inducible promoters, transcription enhancers, transcription terminators, etc., well-known in the art. When two or more nucleic acid molecules are to be co-expressed (e.g., both heavy and light chains of an antibody, or both VH and VL of an antibody), the two nucleic acid molecules may be inserted, for example, into a single expression vector or into separate expression vectors. For single vector expression, the encoding nucleic acids may be operably linked to a common expression control sequence or to different expression control sequences, e.g., one inducible promoter and one constitutive promoter. Methods for confirming the introduction of nucleic acid molecules into host cells that are well-known in the art may be used. It should be understood by those skilled in the art that nucleic acid molecules are expressed in sufficient amounts to produce the desired product (e.g., the anti-Claudin 18.2 antibody as described herein), and it should be further understood that expression levels can be optimized using methods well-known in the art to obtain sufficient expression.

As defined herein, a "subject" refers to any animal (e.g., mammal) that is a recipient of a particular treatment, including, but not limited to, humans, non-human primates, canines, felines, rodents, etc. In some embodiments, the subject is a human. A "subject" may be a patient having a particular disease. In some embodiments, the subject is a patient having a cancer or tumour expressing Claudin 18.2.

As used herein in connection with a disease or condition or a subject having a disease or condition, the term "treating" refers to an action that inhibits, eliminates, alleviates, and/or ameliorates the symptoms, symptom severity, and/or symptom frequency associated with the disease or condition being treated. When used with reference to a cancer or tumour, the term "treating" refers to an action that reduces the severity of the cancer or tumour, or delays or slows the progression of the cancer or tumour, including (a) inhibiting the growth of the cancer or tumour or suppressing the development of the cancer or tumour, or (b) causing regression of the cancer or tumour, or (c) delaying, ameliorating, or minimizing one or more symptoms associated with the presence of the cancer or tumour.

As used herein, the term "administering" refers to the action of delivering or causing the delivery of a therapeutic agent or pharmaceutical composition to an individual's body by the methods described herein or otherwise known in the art. The therapeutic agent may be a compound, a polypeptide, a cell, or a population of cells. Administering a therapeutic agent or pharmaceutical composition includes prescribing the therapeutic agent or pharmaceutical composition to be delivered into a patient's body. Exemplary forms of administration include oral dosage forms such as tablets, capsules, syrups, suspensions; injectable dosage forms such as intravenous (IV), intramuscular (IM), or intraperitoneal (IP); transdermal dosage forms including creams, gels, powders, or patches; buccal dosage forms; inhalable powders, sprays, suspensions; and rectal suppositories.

As used herein, the term "therapeutically effective amount" refers to an amount of a compound, a polypeptide, a cell, a formulation, a substance, or a composition as described herein sufficient to provide a therapeutic benefit in the treatment of a disease or condition or sufficient to delay or minimize one or more symptoms associated with the disease or condition. The disease or condition may be a cancer or tumour expressing Claudin 18.2.

As used herein, the term "carrier" includes "pharmaceutically acceptable carriers", excipients, or stabilizers that are non-toxic to cells or mammals exposed to the carrier at the dosages and concentrations employed. The term "carrier" may also refer to diluents, adjuvants (e.g., Freund's adjuvant (complete and incomplete)), excipients, or vehicles dosed with the therapeutic agent. Examples of suitable pharmaceutical carriers are described in Remington's Pharmaceutical Sciences (1990) Mack Publishing Co., Easton, PA. Compositions comprising pharmaceutical compounds may contain a prophylactically or therapeutically effective amount of, e.g., an anti-β-klotho antibody in an isolated or purified form, along with a suitable amount of carrier to provide a form suitable for dosing to an individual (e.g., a patient). Formulations should be suitable for the mode of dosing.

"Claudin 18.2," "Claudin 18.2," or "CLDN18.2" have the same meaning herein and refer to the type 2 isoform of Claudin 18, a member of the Claudin family of cell surface proteins. Claudins are important components of tight cell junctions, which forms paracellular barriers that control the flow of molecules between cells. Different Claudins are expressed in different tissues, and their altered functions have been associated with cancer formation in these tissues. In normal tissues, the expression of Claudin-18.2 is restricted to epithelial cells of the gastric mucosa. The expression of Claudin 18.2 is maintained after malignant transformation of gastric cancer and its metastases. Ectopic activation of Claudin 18.2 has also been found in pancreatic, oesophageal, ovarian, and lung tumours.

The antibody may be a Fab, Fab', F(ab')2, Fv, scFv, (scFv)$_2$, IgG1 antibody, IgG2 antibody, IgG3 antibody, or IgG4 antibody.

In some embodiments, the Claudin 18.2 binding part includes an antibody. In some embodiments, the Claudin 18.2 binding part includes an antigen-binding fragment of an antibody. In some embodiments, the antibody is an IgA, IgD, IgE, IgG, or IgM antibody. In some embodiments, the antibody is an IgA antibody. In some embodiments, the antibody is an IgD antibody. In some embodiments, the antibody is an IgE antibody. In some embodiments, the antibody is an IgG antibody. In some embodiments, the antibody is an IgM antibody. In some embodiments, the antibody is an IgG1 antibody. In some embodiments, the antibody is an IgG2 antibody. In some embodiments, the antibody is an IgG3 antibody. In some embodiments, the antibody is an IgG4 antibody.

In some embodiments, the Claudin 18.2 binding part includes Fab. In some embodiments, the antibody is Fab'. In some embodiments, the Claudin 18.2 binding part includes F(ab')2. In some embodiments, the Claudin 18.2 binding part includes Fv. In some embodiments, the Claudin 18.2 binding part includes scFv. In some embodiments, the Claudin 18.2 binding part includes disulfide-linked scFv [(scFv)2]. In some embodiments, the Claudin 18.2 binding part includes a diabody (dAb).

In some embodiments, the Claudin 18.2 binding part includes a recombinant antibody. In some embodiments, the Claudin 18.2 binding part includes a monoclonal antibody. In some embodiments, the Claudin 18.2 binding part includes a polyclonal antibody. In some embodiments, the Claudin 18.2 binding part includes a chimeric antibody. In some embodiments, the Claudin 18.2 binding part includes a humanized antibody. In some embodiments, the Claudin 18.2 binding part includes a human antibody.

In some embodiments, the Claudin 18.2 binding part includes a bispecific binding part. In some embodiments, the Claudin 18.2 binding part includes a multispecific binding part.

In some embodiments, the anti-Claudin 18.2 antibody binds to Claudin 18.2 with an affinity at least 20 times greater than its affinity for binding to Claudin 18.1. In some embodiments, the anti-Claudin 18.2 antibody binds to Claudin 18.2 with an affinity at least 50 times greater than its affinity for binding to Claudin 18.1. In some embodiments, the anti-Claudin 18.2 antibody binds to Claudin 18.2 with an affinity at least 100 times greater than its affinity for binding to Claudin 18.1. In some embodiments, the anti-Claudin 18.2 antibody does not detectably bind to Claudin 18.1.

The CDRs of an antibody are defined by those skilled in the art using a variety of methods/systems. These systems and/or definitions have been developed and refined over a number of years and include Kabat, Chothia, IMGT, AbM, and Contact. The Kabat definition is based on sequence variability and is commonly used. The Chothia definition is based on the location of structural loop regions. The IMGT system is based on sequence variability and location within the structure of the variable domain. The AbM definition is a compromise between Kabat and Chothia. The Contact definition is based on analysis of available antibody crystal structures. An exemplary system is the combination of Kabat and Chothia. Software programs for analysing antibody sequences and determining CDRs (e.g., abYsis) are available and known to those skilled in the art.

The specific CDR sequences defined herein are generally based on the Kabat definition. However, it should be understood that reference to one or more heavy chain CDRs and/or one or more light chain CDRs of a particular antibody will encompass all CDR definitions as known to those skilled in the art.

In some embodiments, the Claudin 18.2 binding part is an anti-Claudin 18.2 antibody comprising one, two, three, four, five, and/or six CDRs of any of the antibodies described herein.

In some embodiments, the Claudin 18.2 binding part is a human antibody. Human antibodies can be prepared using various techniques known in the art. In some embodiments, human antibodies are produced by immortalized human B lymphocytes immunized *in vitro*. In some embodiments, human antibodies are produced by lymphocytes isolated from an immunized individual. In any case, cells producing antibodies against the target antigen can be produced and isolated. In some embodiments, human antibodies are selected from phage libraries, wherein the phage libraries express human antibodies. Alternatively, phage display techniques can be used to generate human antibodies and antibody fragments *in vitro* from repertoires of immunoglobulin variable region genes from non-immunized donors. Techniques for the production and use of antibody phage libraries are well-known in the art. Once an antibody is identified, affinity maturation strategies known in the art, including but not limited to chain shuffling and site-directed mutagenesis, can be employed to generate human antibodies having relatively high affinities. In some embodiments, human antibodies are generated in transgenic mice containing human immunoglobulin gene loci. Upon immunization, these mice are capable of producing a full repertoire of human antibodies in the absence of endogenous immunoglobulin production.

Unless otherwise specified, the methods and materials in the examples described below are conventional products that can be purchased from the market. Those skilled in the art to which the present invention belongs will understand that the methods and materials described below are exemplary only and should not be construed as limiting the scope of the present invention.

**Example 1: Obtaining positive clonal molecules of fully human antibodies against human Claudin 18.2**

Screening lead antibody molecules specifically targeting human Claudin 18.2 using a fully human native library: the fully human native library developed independently by Probio Biotech was used, and two screening strategies were employed. One strategy involved 4 rounds of panning (liquid phase panning - cell panning - liquid phase panning - cell panning) using biotinylated Claudin 18.2 proteins for liquid phase panning and positive cells (Human HEK293/Human Claudin 18.2 cell line) and negative cells (HEK293/Human Claudin 18.1 cell line) for cell panning, respectively; and the other strategy involved three rounds of cell panning using positive cells (HEK293/Human Claudin 18.2 cell line) and negative cells (HEK293/Human Claudin 18.1 cell line), obtaining phage library eluates. The neutralized phage panning eluate was added to a TG1 bacterial solution in the logarithmic growth phase. After mixing evenly, the mixture was left to stand at 37°C for 45 minutes to allow the phages to infect the TG1 host bacteria. After sufficient infection, the bacterial suspension was serially diluted, spread onto an agar plate with relevant resistance, and incubated overnight at 37°C in an inverted position. The next day, a 96-well sterile deep well plate was prepared, with each well filled with 0.5 ml of 2YT medium (containing 0.2% w/v of glucose and 0.1 mg/ml of ampicillin antibiotic). Monoclonal colonies cultured in the plate were picked with a sterile pipette tip into the well plate, and the plate was incubated overnight (16-18 hours) at 37°C with shaking at 220 rpm. The following day, 0.05 ml of monoclonal bacterial solution cultured overnight was removed and transferred to a newly prepared sterile deep well plate (filled with 0.45 ml of 2YT medium containing ampicillin antibiotic at a final concentration of 0.1 mg/ml) and cultured until the OD600 value reached approximately 0.6-0.8. 0.5 ml of induction medium (2YT containing 0.1 mg/ml ampicillin antibiotic, 0.2 mM IPTG, 0.2% glycine, 0.04% triton X-100, 0.4% glycerol) was added and incubated in a shaker overnight (16-18 hours) at 30°C with shaking at 220 rpm. The bacterial solution expressed overnight was centrifuged at 4000 rpm for 10 minutes to obtain soluble expression supernatants, which were used to detect binding of monoclones to positive cells (HEK293/Human Claudin 18.2 cell line) and negative cells (HEK293/Human Claudin 18.1 cell line) (Nanjing ProBio) via FACS after panning, so as to obtain positive antibody clones.

FACS assay method: FACS binding assays were used to evaluate the binding capability of antibodies in the supernatant to human Claudin 18.2 and Claudin 18.1 antigens expressed on the membrane surface of HEK293/Human Claudin 18.2 and HEK293/Human Claudin 18.1 cells. HEK293/Human Claudin 18.2 cell lines and HEK293/Human Claudin 18.1 cell lines were collected and washed 3 times with PBS. $1 \times 10^5$ test cells and 50 μl of the supernatant to be tested were added to a 96-well plate, and the plate was incubated at 4°C for 40 minutes. The cells were then washed 3 times with PBS, and 100 μl of Alexa Fluor® 647-labelled goat anti-human IgG F(ab')$_2$ fragment antibody (Jackson, 109-605-006) was added to the well plate, and the plate was incubated at 4°C for 30 minutes. Finally, the cells were washed 3 times with PBS, resuspended in PBS, and the fluorescence signals of the secondary antibody were read using a FACS Calibur (BD).

**Example 2: Sequencing of variable regions of positive clones and recombinant production of monoclonal antibodies**

Based on the FACS assay results, the bacterial solution corresponding to the positive clones were selected for culture, plasmid extraction, PCR and monoclonal Sanger sequencing. Finally, 8 positive clones were obtained. The correspondence between the clones and their sequence numbers is shown in Table 1 below.

Table 1. Positive clones and their corresponding sequence numbers

| Positive clones | Fully human antibody Heavy chain/light chain variable region Amino acid sequence | CDR1 region Amino acid sequence | CDR2 region Amino acid sequence | CDR3 region Amino acid sequence |
|---|---|---|---|---|
| AHP291 53-VH | SEQ ID NO: 1 | SEQ ID NO: 2 | SEQ ID NO: 3 | SEQ ID NO: 4 |
| AHP291 53-VL | SEQ ID NO: 5 | SEQ ID NO: 6 | EVT | SEQ ID NO: 8 |
| AHP308 26-VH | SEQ ID NO: 9 | SEQ ID NO: 10 | SEQ ID NO: 11 | SEQ ID NO: 12 |
| AHP308 26-VL | SEQ ID NO: 13 | SEQ ID NO: 14 | KVS | SEQ ID NO: 16 |
| AHP315 48-VH | SEQ ID NO: 17 | SEQ ID NO: 2 | SEQ ID NO: 3 | SEQ ID NO: 4 |
| AHP315 48-VL | SEQ ID NO: 18 | SEQ ID NO: 19 | EVS | SEQ ID NO: 21 |
| AHP329 13-VH | SEQ ID NO: 22 | SEQ ID NO: 2 | SEQ ID NO: 3 | SEQ ID NO: 4 |
| AHP329 13-VL | SEQ ID NO: 23 | SEQ ID NO: 24 | DTS | SEQ ID NO: 26 |
| AHP329 19-VH | SEQ ID NO: 27 | SEQ ID NO: 28 | SEQ ID NO: 3 | SEQ ID NO: 29 |
| AHP329 19-VL | SEQ ID NO: 30 | SEQ ID NO: 31 | SNN | SEQ ID NO: 33 |
| AHP329 82-VH | SEQ ID NO: 34 | SEQ ID NO: 2 | SEQ ID NO: 3 | SEQ ID NO: 4 |
| AHP329 82-VL | SEQ ID NO: 35 | SEQ ID NO: 36 | AAS | SEQ ID NO: 38 |
| AHP330 07-VH | SEQ ID NO: 34 | SEQ ID NO: 2 | SEQ ID NO: 3 | SEQ ID NO: 4 |
| AHP330 07-VL | SEQ ID NO: 39 | SEQ ID NO: 40 | GAS | SEQ ID NO: 42 |
| AHP330 29-VH | SEQ ID NO: 43 | SEQ ID NO: 28 | SEQ ID NO: 3 | SEQ ID NO: 29 |
| AHP330 29-VL | SEQ ID NO: 44 | SEQ ID NO: 45 | WAS | SEQ ID NO: 47 |

DNA fragments containing the codon-optimized heavy chain, Kappa light chain variable region, and Lambda light chain variable region were synthesized and inserted into huIgG1-opt_pcDNA3.4, hukappa-opt_pcDNA3.4, and hulambda-opt_pcDNA3.4 expression vectors, respectively, to form expression plasmids. These three expression vectors were synthesized by Nanjing ProBio.

The heavy and light chain plasmids were transfected into CHO-S cells according to corresponding clone numbers via the above plasmids, respectively, and the cells were cultured in shake flasks at 37°C for 7 days, and then the supernatant was collected for antibody purification. Prior to purification, the pipelines and the Protein A column were depyrogenated with 0.2 M NaOH. The column was reequilibrated with a buffer containing 0.05 M Tris and 1.5 M NaCl (pH 8.0). Subsequently, the harvested cell culture supernatant was diluted at 1 : 1 with 2 × the above buffer and sterilized by filtration. The filtered supernatant and protein A column were incubated for 2 hours at room temperature. The column was washed with 1 × the above buffer, IgG was then eluted with sterile 0.1 M sodium citrate (pH 3.5); and the eluate was collected and neutralized with one-ninth volume of sterile 1 M Tris-HCl (pH 9.0). Under sterile conditions, the product was buffer-exchanged into PBS (pH 7.4) to remove any elution buffer, and the sample was concentrated. After concentration, the antibodies were quantified at OD280 nm using an extinction coefficient Ec (0.1%) of 1.43.

The purified antibodies were analysed by SDS-PAGE using 10% precast gel (GenScript) and a BioRad electrophoresis system. The gel was stained with Estain 2.0 (GenScript), and the molecular size and purity were estimated by comparing the stained band to Protein Ladder (GenScript).

## Example 3: ELISA binding assay of fully human antibodies to human Claudin 18.2 proteins

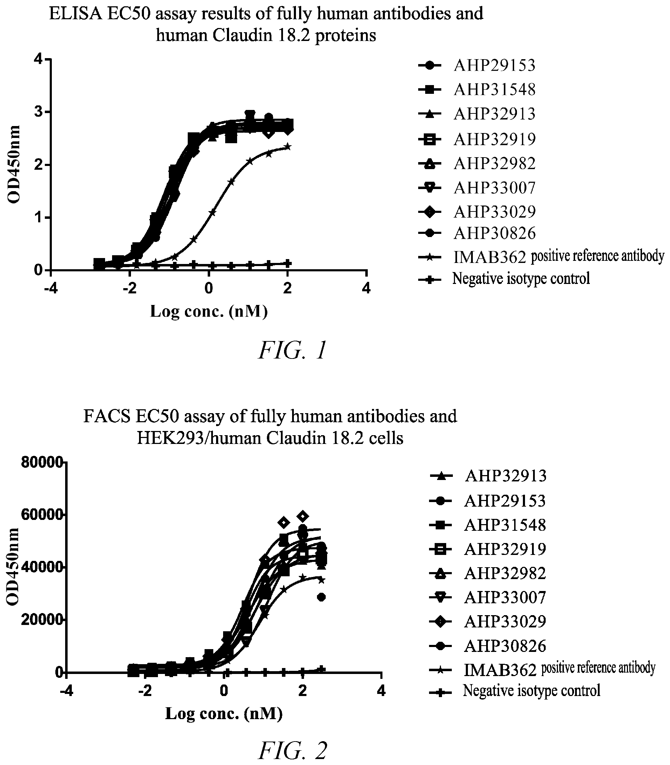

Indirect ELISA was used to assess the binding capability of the above purified antibodies to recombinant human Claudin 18.2 proteins (GenScript, Z03504). The human Claudin 18.2 proteins were diluted to 1 μg/ml with the carbonate buffer, and added to an ELISA plate at 100 μl/well and the plate was coated at 4°C for 16 hours. The plate was washed with PBST (0.05% Tween) and blocked with PBS containing 3% skim milk at 300 μl/well at 37°C for 1 hour. Subsequently, the blocking solution was discarded, and 100 μl of purified antibody starting from 15 μg/ml (approx. 100 nM) in the first well with 3-fold gradient dilutions was added, for a total of 11 test concentration gradients. PBST (0.05% Tween) was added to the last well. The plate was incubated in an oven at 37°C for 1 hour. The plate was washed 3 times with PBST and horseradish peroxidase-conjugated mouse anti-human IgG Fc fragment (GenScript, A01854) was added at 100 μl/well and incubated at 37°C for 0.5 hours. The plate was washed 6 times with PBST, a TMB colour developing solution (GenScript) was then added, and the plate was incubated for 10 minutes at room temperature in the dark. The reaction was stopped by adding 50 μl of 1M HCl stop solution (Sigma), and the plate was read at 450 nm using a microplate reader. The ELISA binding results of the 8 positive recombinant antibodies to human Claudin 18.2 proteins are shown in FIG. 1. The specific $EC_{50}$ for each antibody is shown in Table 2 below. Compared to the positive reference antibody Zolbetuximab (IMAB362, HuIgG1), the binding capabilities of these tested antibodies to the human Claudin 18.2 antigen proteins were significantly better than that of the positive reference antibody.

**Table 2. ELISA $EC_{50}$ assay of purified antibodies and human Claudin 18.2-related proteins**

| Purified antibody | Human Claudin 18.2 protein ELISA $EC_{50}$ (nM) |
|---|---|
| AHP29153 | 0.1251 |
| AHP31548 | 0.07402 |
| AHP32913 | 0.111 |
| AHP32919 | 0.07878 |
| AHP32982 | 0.1194 |
| AHP33007 | 0.139 |
| AHP33029 | 0.07457 |
| AHP30826 | 0.09142 |
| IMAB362 positive reference antibody | 1.532 |

**Example 4: FACS $EC_{50}$ of fully human antibodies for HEK293 cell lines expressing human and murine Claudin 18.2, and human Claudin 18.1**

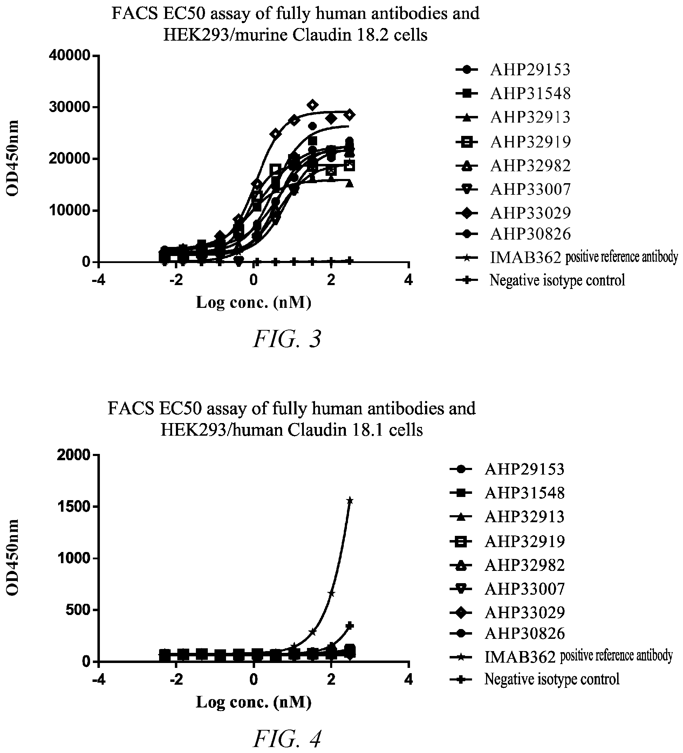

HEK-293 cell lines expressing human and murine Claudin 18.2, and human Claudin 18.1 for testing were collected and washed 3 times with PBS. $1 \times 10^5$ test cells and 100 μl of purified antibody starting from 45 μg/ml (approx. 300 nM) in the first well with 3-fold gradient dilutions, for a total of 11 test concentration gradients, were added to a 96-well plate, and the plate was incubated at 4°C for 45 minutes. The cells were then washed 3 times with PBS, 100 μl of FITC-labelled goat anti-human IgG F(ab')$_2$ fragment antibody was added, and the plate was incubated at 4°C for 30 minutes. Finally, the cells were washed 3 times with PBS, and the signals were read using FACS BD Calibur. As shown in FIGS. 2-4, the antibodies bound to both HEK-293 cells expressing human Claudin 18.2 and HEK-293 cells expressing murine Claudin 18.2, but did not bind to HEK-293 cells expressing human Claudin 18.1. The specific FACS $EC_{50}$ for each antibody is shown in Table 3 below. Compared to the positive reference antibody IMAB362, the tested antibodies showed cross-species reactivity with murine Claudin 18.2. The binding capabilities of the tested antibodies to HEK-293 cells expressing human and murine Claudin 18.2 were stronger than or comparable to that of the positive reference antibody, and the tested antibodies showed no binding to HEK-293 cells expressing human Claudin 18.1.

**Table 3. FACS $EC_{50}$ assay of fully human antibodies and Claudin 18.2 and Claudin 18.1 related cells**

| Purified antibody | HEK293/human Claudin 18.2 FACS $EC_{50}$ (nM) | HEK293/murine Claudin 18.2 FACS $EC_{50}$ (nM) | HEK293/human Claudin 18.1 protein FACS $EC_{50}$ (nM) |
|---|---|---|---|
| AHP29153 | 8.149 | 4.452 | NA |
| AHP31548 | 3.076 | 1.671 | NA |
| AHP32913 | 4.159 | 0.7281 | NA |
| AHP32919 | 6.041 | 0.7718 | NA |
| AHP32982 | 5.91 | 4.03 | NA |
| AHP33007 | 12.45 | 7.219 | NA |
| AHP33029 | 4.235 | 1.203 | NA |

(continued)

| Purified antibody | HEK293/human Claudin 18.2 FACS $EC_{50}$ (nM) | HEK293/murine Claudin 18.2 FACS $EC_{50}$ (nM) | HEK293/human Claudin 18.1 protein FACS $EC_{50}$ (nM) |
|---|---|---|---|
| AHP30826 | 3.535 | 3.437 | NA |
| IMAB362 positive reference antibody | 7.805 | 3.968 | NA |

**Example 5: SPR binding assay of fully human antibodies to human Claudin 18.2 proteins**

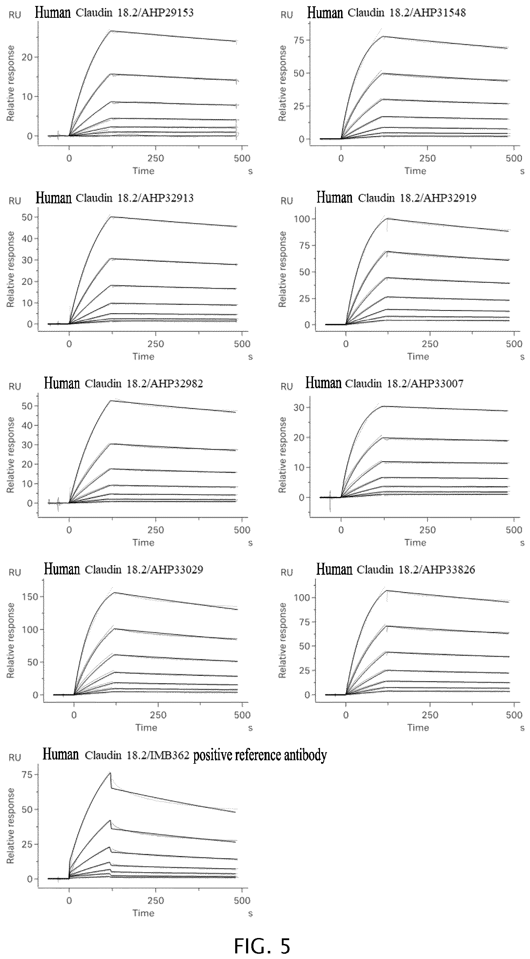

Surface plasmon resonance (SPR) biosensor Biacore 8K (Cytiva) was used to determine the affinity of purified antibodies for human Claudin 18.2 proteins. The antibodies were immobilized on the sensor chip using the Fc capture method. Human Claudin 18.2 proteins were used as analytes. Data for dissociation (kd) and association (ka) rate constants were obtained using the Biacore 8K evaluation software, and the equilibrium dissociation constant (KD) was calculated as the ratio of kd to ka. The results of the SPR affinity assay are as shown in Table 4 below, and the relevant sensorgrams are as shown in FIG. 5. The tested antibodies and the positive reference antibody IMAB362 exhibited comparable affinity levels for the human Claudin 18.2 antigen proteins, with their equilibrium dissociation constants (KD) all in the range of $10^{-8}$.

**Table 4. Affinity determination of humanized fully human antibodies for human Claudin 18.2 proteins**

| Ligand | Analyte | KD (M) |
|---|---|---|
| AHP29153 | Human Claudin 18.2 | 9.96E-09 |
| AHP31548 | Human Claudin 18.2 | 7.31E-09 |
| AHP32913 | Human Claudin 18.2 | 9.44E-09 |
| AHP32919 | Human Claudin 18.2 | 7.91E-09 |
| AHP32982 | Human Claudin 18.2 | 1.33E-08 |
| AHP33007 | Human Claudin 18.2 | 2.70E-09 |
| AHP33029 | Human Claudin 18.2 | 1.02E-08 |
| AHP30826 | Human Claudin 18.2 | 7.82E-09 |
| IMAB362 positive reference antibody | Human Claudin 18.2 | 3.93E-08 |

**Example 6: Identification of binding epitopes of fully human antibodies to human Claudin 18.2 proteins**

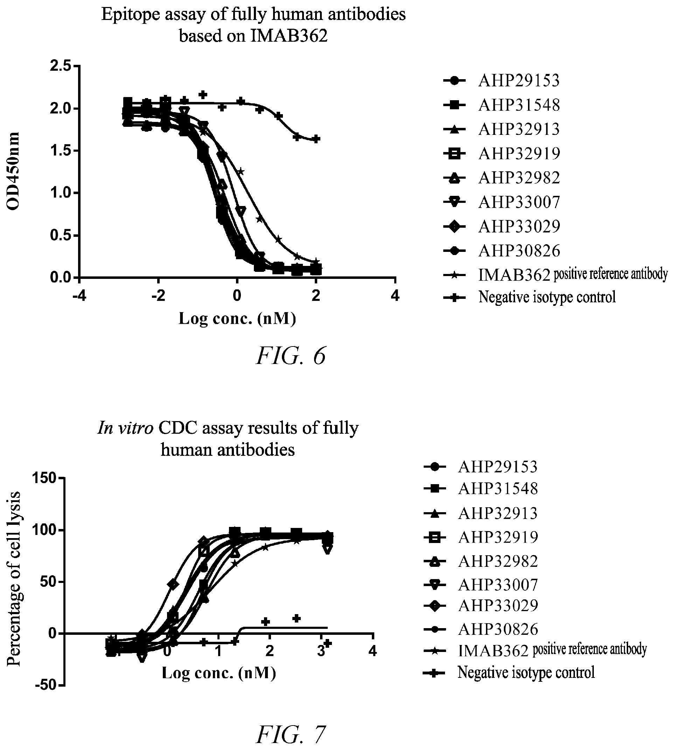

Indirect ELISA was used to evaluate whether the binding epitopes of the above purified antibodies to human Claudin 18.2 proteins were the same as those of the positive reference antibody IMAB362. The ELISA plate was coated with 1 μg/ml recombinant human Claudin 18.2 protein in CBS buffer at 100 μl/well at 4°C overnight. The plate was washed with PBST (0.05% Tween) and blocked with PBS containing 3% skim milk at 300 μl/well at 37°C for 1 hour. Subsequently, the blocking solution was discarded, and 50 μl of purified antibody (starting from 30 μg/ml (approx. 200 nM) in the first well with 3-fold gradient dilutions, a total of 11 test concentration gradients) was added, and incubated at 37°C for 1 hour. Then, 50 μl of 0.72 μg/ml biotinylated positive reference antibody was added and incubated at 37°C for 1 hour. The plate was washed 3 times with PBST and horseradish peroxidase-conjugated streptavidin antibody (GenScript) was added at 100 μl/well and incubated at 37°C for 0.5 hours. The plate was washed 6 times with PBST, a TMB colour developing solution (GenScript) was then added, and the plate was incubated for 10 minutes at room temperature in the dark. The reaction was stopped by adding 50 μl of 1M HCl stop solution (Sigma), and the plate was read at 450 nm using a microplate reader. The experiment results of the assay for binding epitopes of fully human antibodies to human Claudin 18.2 proteins are shown in FIG. 6. The specific $IC_{50}$ for each antibody is shown in Table 5 below. The binding epitopes of the tested antibodies to human Claudin 18.2 proteins were the same as those of the positive reference antibody IMAB362.

**Table 5. ELISA $IC_{50}$ assay of purified antibodies and human Claudin 18.2-related proteins**

| Purified antibody | ELISA $IC_{50}$ (nM) for binding epitopes compared to IMAB362 |
|---|---|
| AHP29153 | 0.3227 |
| AHP31548 | 0.2649 |
| AHP32913 | 0.3368 |

(continued)

| Purified antibody | ELISA IC$_{50}$ (nM) for binding epitopes compared to IMAB362 |
|---|---|
| AHP32919 | 0.2707 |
| AHP32982 | 0.4907 |
| AHP33007 | 0.8054 |
| AHP33029 | 0.3185 |
| AHP30826 | 0.2758 |
| IMAB362 positive reference antibody | 1.793 |

**Example 7: *In vitro* CDC test of fully human antibodies targeting human Claudin 18.2**

The CHO-K1/human Claudin 18.2 cell suspension was adjusted to an appropriate plating density (5000 cells/well) using CDC buffer. The target cell suspension was added to the corresponding wells of a 384-well assay plate, followed by addition of control working solution, sample working solution, and CDC buffer and mixing well, then the plate was incubated in a cell incubator (37°C, 5% $CO_2$) for about 30 minutes. The mixed NHSC was diluted with CDC buffer to prepare its working solution (at a concentration 4 times the final concentration to be tested). After the completion of incubation, the NHSC working solution was added to the corresponding wells of the 384-well assay plate. After mixing well, the plate was transferred to a cell incubator (37°C, 5% CO2) and incubated for about 4 hours. The Cell Titre-Glo Luminescent working solution was taken out and allowed to reach room temperature. The 384-well assay plate was removed. According to experimental requirements, 30 $\mu$l of Cell titre-Glo Luminescent working solution was added to each well and mixed well, then the plate was incubated at room temperature for 5-10 minutes. Luminescence signal values were read using a microplate reader, and the percentage of cell lysis caused by CDC was calculated. The results showed that: the CDC activities of the tested molecules were either superior to or comparable to that of the positive reference. Among them, AHP33029 exhibited the best CDC activity. Detailed results are shown in Table 6 and FIG. 7.

The calculation formula is as follows:

$$\text{Cell Lysis} \% = 100\% \times (1 - (\text{RLU}_{\text{Sample}} - \text{RLU}_{\text{NHSC}})/(\text{RLU}_{\text{cell+NHSC}} - \text{RLU}_{\text{NHSC}}))$$

Note: Sample group = target cells + test sample + NHSC working solution, used to detect the complement killing signal mediated by the sample; Cell + NHSC group = target cells + NHSC working solution, used to detect the signal level under natural lysis of all target cells;
NHSC group = only NHSC working solution, used to detect the background signal value of NHSC.

Table 6. *In vitro* CDC assay results of fully human antibodies targeting human Claudin 18.2

| ID | IMAB36 2 positive reference antibody | AHP 2915 3 | AHP 3154 8 | AHP 3291 3 | AHP 3291 9 | AH P32 982 | AH P33 007 | AH P33 029 | AHP 3082 6 | Hum an IgG1 |
|---|---|---|---|---|---|---|---|---|---|---|
| Top | 93.05 | 96.31 | 93.97 | 92.98 | 96.29 | 95.5 2 | 92.7 4 | 96.8 9 | 96.02 | 5.608 |
| EC$_{50}$ | 6.951 | 2.496 | 3.980 | 2.024 | 2.260 | 5.79 7 | 4.61 8 | 1.11 5 | 2.242 | ~ 23.72 |

**Example 8: *In vitro* ADCC test of fully human antibodies targeting human Claudin 18.2**

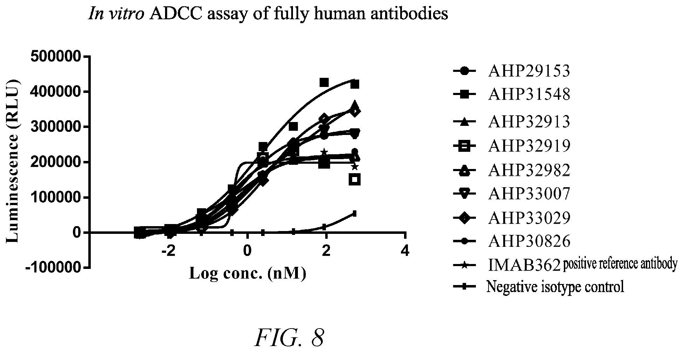

Cells were taken out from the carbon dioxide incubator, cell status was observed under an inverted microscope, and then the cells were transferred to a biosafety cabinet that had been UV-irradiated for more than 30 minutes. Medium was discarded, and the cells were washed once with 4-6 ml of DPBS. 1-2 ml of Accutase was added to digest the cells. When the cells became rounded and detached, the cells could naturally detach under gently shaking, and digestion was terminated by adding 4-6 ml of complete medium. The cell suspension was collected into a centrifuge tube and centrifuged at 125 $\times$ g for 5 minutes. After centrifugation, the supernatant was discarded, and cells were resuspended in a complete medium, mixed well, and counted. According to the number of samples, a target cell suspension of appropriate volume and defined density was formulated using a complete medium. A certain amount of target cell CHO-K1/Claudin 18.2 suspension was transferred to the corresponding wells of a 96-well assay plate. During transfer, the target cells was pipetted to keep the cell suspension remain in a homogeneous state. Control/sample working solutions at different concentrations were transferred to the corresponding wells of the assay plate according to the experimental layout, and the plate was incubated at room temperature for 30 minutes. Effector cells GS-J2C/CD16A 158V were collected and resuspended in an assay buffer, mixed well, and counted. According to the number of samples, an effector cell suspension of appropriate volume and defined density was formulated using an assay buffer. A certain amount of effector cell suspension was transferred to the corresponding wells of a 96-well assay plate. During transfer, the cells was pipetted to keep the cell suspension remain in a homogeneous state. The assay plate was transferred to the carbon dioxide incubator (37°C, 5% $CO_2$) and incubated for 6 hours. After the completion of incubation, the assay plate was taken out and equilibrated to room temperature. According to the experimental layout, a certain volume of fluorescent detection reagent was added to each well, and the plate was incubated at room temperature for 5-10 minutes in the dark. Chemiluminescence signal values were then read using a microplate reader. The results showed that: the activities of the tested molecules in the ADCC reporter gene assay were either superior to or comparable to that of the positive reference. Detailed results are shown in Table 7 and FIG. 8.

The calculation formula is as follows:

$$Y = \text{Bottom} + (\text{Top-Bottom}) / (1 + 10^{((\text{LogEC}_{50} - X) \times \text{HillSlope}))}$$

X = Independent variable
Bottom = Minimum dose response
HillSlope = Curvature parameter hillslope
$\text{LogEC}_{50}$ = Half-dose concentration $EC_{50}$
Top = Maximum dose response

**Table 7.** *In vitro* ADCC assay results of fully human antibodies targeting human Claudin 18.2

| ID | IMAB362 Positive reference antibody | AHP 29153 | AHP 31548 | AHP 32913 | AHP 32919 | AH P33 029 | AH P33 007 | AH P32 982 | AH P30 826 | Human IgG1 |
|---|---|---|---|---|---|---|---|---|---|---|
| **Top** | 213253 | 28286 1 | 46340 7 | 48567 3 | 19894 4 | 357 815 | 295 058 | 216 188 | 222 023 | 9052 2 |
| **EC$_{50}$ (nM)** | 0.7583 | 0.805 3 | 2.149 | 8.123 | ~ 0.439 1 | 3.73 3 | 1.84 3 | 0.3 789 | 0.6 842 | 352.3 |

The information of the amino acid and nucleotide sequences mentioned herein is as follows:

The amino acid sequence of AHP29153-VH fully human antibody variable region:

SEQ ID NO: 1

QLQQVESGGGLVQPGGSLRLSCAASRNIGILSTMGWYRQAPGKQRELVAT
ITRGGSTNYGEFVKGRFTISRDNAKNTVYLQMNSLKPEDTAVYYCNVDES
WLGTQSYWGRGTTVTVSS

The amino acid sequence of AHP29153-VH CDR1 region: SEQ ID NO: 2
RNIGILST
The amino acid sequence of AHP29153-VH CDR2 region: SEQ ID NO: 3
ITRGGST
The amino acid sequence of AHP29153-VH CDR3 region: SEQ ID NO: 4
NVDESWLGTQSY
The amino acid sequence of AHP29153-VL fully human antibody variable region:

SEQ ID NO: 5

QSVLTQPASVSGSPGQSITISCTGTSSDVGGYKYVSWYQQPPGKAPKLIIYE
VTKRPSGVPDRFSGSKSGNTASLTVSGLQAEDEADYYCLSYAGSNNVFGT
GTKLTVL

The amino acid sequence of AHP29153-VL CDR1 region: SEQ ID NO: 6
SSDVGGYKY
The amino acid sequence of AHP29153-VL CDR2 region: SEQ ID NO: 7
EVT
The amino acid sequence of AHP29153-VL CDR3 region: SEQ ID NO: 8
LSYAGSNNV
The amino acid sequence of AHP30826-VH fully human antibody variable region:

SEQ ID NO: 9

QVQLQQSGGGSVQAGGSLRLSCAASGTILSINAMGWYRQAPGKQRELVA
TITTGGNANYAHSVKGRFTISRDNAKNTVYLQMNNLKPEDTAVYYCNAE
QVVVGFLQAYDVWGQGTTVTVSS

The amino acid sequence of AHP30826-VH CDR1 region: SEQ ID NO: 10
GTILSINA
The amino acid sequence of AHP30826-VH CDR2 region: SEQ ID NO: 11
ITTGGNA
The amino acid sequence of AHP30826-VH CDR3 region: SEQ ID NO: 12
NAEQVVVGFLQAYDV
The amino acid sequence of AHP30826-VL fully human antibody variable region:

SEQ ID NO: 13

DVVMTQSPLSLPVTLGQAASISCRSSQSLVHGDGNTYLNWIQQRPGQSPRR
LIYKVSNRDSGVPDRFSGSGSGTDFTLKISRVEAEDVGVYYCMQGTHWPS
TFGPGTKVDIK

The amino acid sequence of AHP30826-VL CDR1 region: SEQ ID NO: 14
QSLVHGDGNTY
The amino acid sequence of AHP30826-VL CDR2 region: SEQ ID NO: 15
KVS
The amino acid sequence of AHP30826-VL CDR3 region: SEQ ID NO: 16
MQGTHWPST
The amino acid sequence of AHP31548-VH fully human antibody variable region:

SEQ ID NO: 17

QVQLMQSGGGLVQPGGSLRLSCAASRNIGILSTMGWYRQAPGKQRELVA
TITRGGSTNYGEFVKGRFTISRDNAKNTVYLQMNSLKPEDTAVYYCNVDE
SWLGTQSYWGRGTTVTVSS

The amino acid sequence of AHP31548-VH CDR1 region: SEQ ID NO: 2
RNIGILST
The amino acid sequence of AHP31548-VH CDR2 region: SEQ ID NO: 3
ITRGGST
The amino acid sequence of AHP31548-VH CDR3 region: SEQ ID NO: 4
NVDESWLGTQSY
The amino acid sequence of AHP31548-VL fully human antibody variable region:

SEQ ID NO: 18

QSALTQPPSASGSPGQSVTISCTGTSSDIGTYNFVSWYQQHPGKAPKLIIYE
VSKRPSGVPDRFSGSKSGNTASLTVSGLQAEDEADYYCKSYPRVFGTGTK
LTAL

The amino acid sequence of AHP31548-VL CDR1 region: SEQ ID NO: 19
SSDIGTYNF
The amino acid sequence of AHP31548-VL CDR2 region: SEQ ID NO: 20
EVS
The amino acid sequence of AHP31548-VL CDR3 region: SEQ ID NO: 21
KSYPRV
The amino acid sequence of AHP32913-VH fully human antibody variable region:

SEQ ID NO: 22

QVQLMQSGGGLVQPGGSLRLSCAASRNIGILSTMGWYRQAPGKQRELVA
TITRGGSTNYGEFVKGRFTISRDNAKNTVYLQMNSLKPEDTAVYYCNVDE
SWLGTQSYWGRGTQVTVSS

The amino acid sequence of AHP32913-VH CDR1 region: SEQ ID NO: 2
RNIGILST
The amino acid sequence of AHP32913-VH CDR2 region: SEQ ID NO: 3
ITRGGST
The amino acid sequence of AHP32913-VH CDR3 region: SEQ ID NO: 4
NVDESWLGTQSY
The amino acid sequence of AHP32913-VL fully human antibody variable region:

SEQ ID NO: 23

QAVVTQEPSLTVSPGGTVTLTCGSSTGPVTSGHYPHWYQQKPGQAPKTLIF

DTSHKHPWTPAQFSGSLLGGKAALTLSGAHPEDEAEYYCLLSYSGVRVFG

GGTQLIIL

The amino acid sequence of AHP32913-VL CDR1 region: SEQ ID NO: 24
TGPVTSGHY
The amino acid sequence of AHP32913-VL CDR2 region: SEQ ID NO: 25
DTS
The amino acid sequence of AHP32913-VL CDR3 region: SEQ ID NO: 26
LLSYSGVRV
The amino acid sequence of AHP32919-VH fully human antibody variable region:

SEQ ID NO: 27

QVQLQESGGGVVQPGGSLRLSCAASQSIGLLSTMGWYRQAAGKQRELVA

TITRGGSTNYGEFVKGRFTISRDNAKNTVYLQMNSLKPEDTAVYYCTADE

SWLGTQSYWGRGTRVTVSS

The amino acid sequence of AHP32919-VH CDR1 region: SEQ ID NO: 28
QSIGLLST
The amino acid sequence of AHP32919-VH CDR2 region: SEQ ID NO: 3
ITRGGST
The amino acid sequence of AHP32919-VH CDR3 region: SEQ ID NO: 29
TADESWLGTQSY
The amino acid sequence of AHP32919-VL fully human antibody variable region:

SEQ ID NO: 30

QAGLTQPPSASGTPGQGVTISCSGSSSNIGSNSVNWYQQLPGTAPKLLMHS

NNERSSGVPDRFSGSKSGSSASLAISGLQSEDAADYFCGAWDDSLNGYVF

GTGTKLTVL

The amino acid sequence of AHP32919-VL CDR1 region: SEQ ID NO: 31
SSNIGSNS
The amino acid sequence of AHP32919-VL CDR2 region: SEQ ID NO: 32
SNN
The amino acid sequence of AHP32919-VL CDR3 region: SEQ ID NO: 33
GAWDDSLNGYV
The amino acid sequence of AHP32982-VH fully human antibody variable region:

SEQ ID NO: 34

QVQLVQSGGGLVQPGGSLRLSCAASRNIGILSTMGWYRQAPGKQRELVAT

ITRGGSTNYGEFVKGRFTISRDNAKNTVYLQMNSLKPEDTAVYYCNVDES

WLGTQSYWGRGPLVTVSS

The amino acid sequence of AHP32982-VH CDR1 region: SEQ ID NO: 2
RNIGILST
The amino acid sequence of AHP32982-VH CDR2 region: SEQ ID NO: 3
ITRGGST
The amino acid sequence of AHP32982-VH CDR3 region: SEQ ID NO: 4
NVDESWLGTQSY

The amino acid sequence of AHP32982-VL fully human antibody variable region:

SEQ ID NO: 35

DIVMTQSPSSVSASVGDRVTITCRASQGISSWLAWYQQKPGKAPKLLIYAASSLQSGVPSRFSGSGSGTDFTLTISSLQPEDFATYYCQQANSFPLTFGGGTKVEIK

The amino acid sequence of AHP32982-VL CDR1 region: SEQ ID NO: 36
QGISSW
The amino acid sequence of AHP32982-VL CDR2 region: SEQ ID NO: 37
AAS
The amino acid sequence of AHP32982-VL CDR3 region: SEQ ID NO: 38
QQANSFPLT
The amino acid sequence of AHP33007-VH fully human antibody variable region:

SEQ ID NO: 34

QVQLVQSGGGLVQPGGSLRLSCAASRNIGILSTMGWYRQAPGKQRELVATITRGGSTNYGEFVKGRFTISRDNAKNTVYLQMNSLKPEDTAVYYCNVDESWLGTQSYWGRGPLVTVSS

The amino acid sequence of AHP33007-VH CDR1 region: SEQ ID NO: 2
RNIGILST
The amino acid sequence of AHP33007-VH CDR2 region: SEQ ID NO: 3
ITRGGST
The amino acid sequence of AHP33007-VH CDR3 region: SEQ ID NO: 4
NVDESWLGTQSY
The amino acid sequence of AHP33007-VL fully human antibody variable region:

SEQ ID NO: 39

EIVMTQSPATLSVSPGERATLSCRASQSVSSNLAWYQQKPGQAPRLLIYGASTRATGIPARFSGSGSGTEFTLTISSLQSEDFAVYYCQQYNNWPPWTFGQGTKVEIK

The amino acid sequence of AHP33007-VL CDR1 region: SEQ ID NO: 40
QSVSSN
The amino acid sequence of AHP33007-VL CDR2 region: SEQ ID NO: 41
GAS
The amino acid sequence of AHP33007-VL CDR3 region: SEQ ID NO: 42
QQYNNWPPWT
The amino acid sequence of AHP33029-VH fully human antibody variable region:

SEQ ID NO: 43

QVQLVDSGGGLVQSGGSLRLTCAASQSIGLLSTMGWYRQAAGKQRELVATITRGGSTNYGEFVKGRFTISRDNAKNTVYLQMNSLKPEDTAVYYCTADESWLGTQSYWGRGTLVTVSS

The amino acid sequence of AHP33029-VH CDR1 region: SEQ ID NO: 28
QSIGLLST

The amino acid sequence of AHP33029-VH CDR2 region: SEQ ID NO: 3
ITRGGST
The amino acid sequence of AHP33029-VH CDR3 region: SEQ ID NO: 29
TADESWLGTQSY
The amino acid sequence of AHP33029-VL fully human antibody variable region:

SEQ ID NO: 44

DIVMTQSPDSLAVSLGERATINCKSSQSVLYSSNNKNYLAWYQQKPGQPP
KLLIYWASTRESGVPDRFSGSGSGTDFTLTISSLQAEDVAVYYCQQYYSTP
RTFGQGTKVEIK

The amino acid sequence of AHP33029-VL CDR1 region: SEQ ID NO: 45
QSVLYSSNNKNY
The amino acid sequence of AHP33029-VL CDR2 region: SEQ ID NO: 46
WAS
The amino acid sequence of AHP33029-VL CDR3 region: SEQ ID NO: 47
QQYYSTPRT

## Claims

1. A binding part for specifically binding to Claudin 18.2, **characterized in that** the binding part for specifically binding to Claudin 18.2 comprises

   a heavy chain variable region (VH), wherein the VH comprises: a VH CDR1, a VH CDR2, and a VH CDR3; wherein the VH CDR1, the VH CDR2, and the VH CDR3 comprise:

   (1) amino acid sequences as set forth in SEQ ID NOs: 2, 3, and 4, respectively;
   (2) amino acid sequences as set forth in SEQ ID NOs: 10, 11, and 12, respectively; or
   (3) amino acid sequences as set forth in SEQ ID NOs: 28, 3, and 29, respectively,

   or a variant thereof comprising up to 3 amino acid substitutions in the CDRs; and
   (b) a light chain variable region (VL), wherein the VL comprises: a VL CDR1, a VL CDR2, and a VL CDR3; wherein the VL CDR1, the VL CDR2, and the VL CDR3 comprise:

   (1) an amino acid sequence as set forth in SEQ ID NO: 6, EVT, and an amino acid sequence as set forth in SEQ ID NO: 8, respectively;
   (2) an amino acid sequence as set forth in SEQ ID NO: 14, KVS, and an amino acid sequence as set forth in SEQ ID NO: 16, respectively;
   (3) an amino acid sequence as set forth in SEQ ID NO: 19, EVS, and an amino acid sequence as set forth in SEQ ID NO: 21, respectively;
   (4) an amino acid sequence as set forth in SEQ ID NO: 24, DTS, and an amino acid sequence as set forth in SEQ ID NO: 26, respectively;
   (5) an amino acid sequence as set forth in SEQ ID NO: 31, SNN, and an amino acid sequence as set forth in SEQ ID NO: 33, respectively;
   (6) an amino acid sequence as set forth in SEQ ID NO: 36, AAS, and an amino acid sequence as set forth in SEQ ID NO: 38, respectively;
   (7) an amino acid sequence as set forth in SEQ ID NO: 40, GAS, and an amino acid sequence as set forth in SEQ ID NO: 42, respectively; or
   (8) an amino acid sequence as set forth in SEQ ID NO: 45, WAS, and an amino acid sequence as set forth in SEQ ID NO: 47, respectively,

   or a variant thereof comprising up to 3 amino acid substitutions in the CDRs.

2. The binding part according to claim 1, **characterized in that**

the VH CDR1, the VH CDR2, and the VH CDR3 comprise the amino acid sequences as set forth in SEQ ID NOs: 2, 3, and 4, respectively, and the VL CDR1, the VL CDR2, and the VL CDR3 comprise the amino acid sequence as set forth in SEQ ID NO: 6, EVT, and the amino acid sequence as set forth in SEQ ID NO: 8, respectively;

the VH CDR1, the VH CDR2, and the VH CDR3 comprise the amino acid sequences as set forth in SEQ ID NOs: 2, 3, and 4, respectively, and the VL CDR1, the VL CDR2, and the VL CDR3 comprise the amino acid sequence as set forth in SEQ ID NO: 19, EVS, and the amino acid sequence as set forth in SEQ ID NO: 21, respectively;

the VH CDR1, the VH CDR2, and the VH CDR3 comprise the amino acid sequences as set forth in SEQ ID NOs: 2, 3, and 4, respectively, and the VL CDR1, the VL CDR2, and the VL CDR3 comprise the amino acid sequence as set forth in SEQ ID NO: 24, DTS, and the amino acid sequence as set forth in SEQ ID NO: 26, respectively;

the VH CDR1, the VH CDR2, and the VH CDR3 comprise the amino acid sequences as set forth in SEQ ID NOs: 2, 3, and 4, respectively, and the VL CDR1, the VL CDR2, and the VL CDR3 comprise the amino acid sequence as set forth in SEQ ID NO: 36, AAS, and the amino acid sequence as set forth in SEQ ID NO: 38, respectively;

the VH CDR1, the VH CDR2, and the VH CDR3 comprise the amino acid sequences as set forth in SEQ ID NOs: 2, 3, and 4, respectively, and the VL CDR1, the VL CDR2, and the VL CDR3 comprise the amino acid sequence as set forth in SEQ ID NO: 40, GAS, and the amino acid sequence as set forth in SEQ ID NO: 42, respectively;

the VH CDR1, the VH CDR2, and the VH CDR3 comprise the amino acid sequences as set forth in SEQ ID NOs: 10, 11, and 12, respectively, and the VL CDR1, the VL CDR2, and the VL CDR3 comprise the amino acid sequence as set forth in SEQ ID NO: 14, KVS, and the amino acid sequence as set forth in SEQ ID NO: 16, respectively;

the VH CDR1, the VH CDR2, and the VH CDR3 comprise the amino acid sequences as set forth in SEQ ID NOs: 28, 3, and 29, respectively; and the VL CDR1, the VL CDR2, and the VL CDR3 comprise the amino acid sequence as set forth in SEQ ID NO: 31, SNN, and the amino acid sequence as set forth in SEQ ID NO: 33, respectively; or

the VH CDR1, the VH CDR2, and the VH CDR3 comprise the amino acid sequences as set forth in SEQ ID NOs: 28, 3, and 29, respectively; and the VL CDR1, the VL CDR2, and the VL CDR3 comprise the amino acid sequence as set forth in SEQ ID NO: 45, WAS, and the amino acid sequence as set forth in SEQ ID NO: 47, respectively.

3. The binding part according to claim 1 or 2, **characterized in that** the VL is a Kappa light chain variable region or a Lambda light chain variable region.

4. The binding part according to claim 3, **characterized in that** the VH comprises an amino acid sequence having at least 80% sequence identity to an amino acid sequence selected from the group consisting of: SEQ ID NOs: 1, 9, 17, 22, 27, 34, and 43; or the VL comprises an amino acid sequence having at least 80% sequence identity to an amino acid sequence selected from the group consisting of: SEQ ID NOs: 5, 13, 18, 23, 30, 35, 39, and 44.

5. The binding part according to claim 4, **characterized in that** the VH and VL comprise:

(1) the amino acid sequences as set forth in SEQ ID NOs: 1 and 5, respectively;
(2) the amino acid sequences as set forth in SEQ ID NOs: 9 and 13, respectively;
(3) the amino acid sequences as set forth in SEQ ID NOs: 17 and 18, respectively;
(4) the amino acid sequences as set forth in SEQ ID NOs: 22 and 23, respectively;
(5) the amino acid sequences as set forth in SEQ ID NOs: 27 and 30, respectively;
(6) the amino acid sequences as set forth in SEQ ID NOs: 34 and 35, respectively;
(7) the amino acid sequences as set forth in SEQ ID NOs: 34 and 39, respectively; or
(8) the amino acid sequences as set forth in SEQ ID NOs: 43 and 44, respectively.

6. The binding part according to any one of claims 1-5, **characterized in that** the binding part is sdab, HcAb, Fab, Fab', $F(ab')_2$, Fv, scFv, $(scFv)_2$, or a full-length antibody.

7. The binding part according to claim 1, **characterized in that** the binding part is a mouse binding part, a chimeric binding part, a humanized binding part, or a fully human binding part.

8. The binding part according to claim 7, **characterized in that** the binding part is fully human.

9. The binding part according to claim 6, **characterized in that** the binding part is a fully human full-length antibody.

10. A multispecific antibody or a binding fragment thereof, **characterized in that** the multispecific antibody or the binding fragment thereof comprises the binding part according to any one of claims 1-9.

11. A chimeric antigen receptor, **characterized in that** the chimeric antigen receptor comprises:

    (a) an extracellular antigen-binding domain, comprising the binding part according to any one of claims 1-9, or the multispecific antibody or the binding fragment thereof according to claim 10;
    (b) a transmembrane domain; and
    (c) an intracellular signalling domain.

12. A nucleic acid, **characterized in that** the nucleic acid encodes the binding part according to any one of claims 1-9, the multispecific antibody or the binding fragment thereof according to claim 10, or the chimeric antigen receptor according to claim 11.

13. A host cell, **characterized in that** the host cell comprises the nucleic acid according to claim 12.

14. A pharmaceutical composition, **characterized in that** the pharmaceutical composition comprises the binding part according to any one of claims 1-9, the multispecific antibody or the binding fragment thereof according to claim 10, the chimeric antigen receptor according to claim 11, or the host cell according to claim 13, and a pharmaceutically acceptable carrier.

15. A method for treating a disease associated with the expression of Claudin 18.2 in a subject in need thereof, **characterized in that** the method comprises administering to the subject a therapeutically effective amount of the pharmaceutical composition according to claim 14.

16. The method according to claim 15, **characterized in that** the disease associated with the expression of Claudin 18.2 is gastric cancer, pancreatic cancer, oesophageal cancer, breast cancer, colon cancer, liver cancer, ovarian cancer, or lung cancer.

17. The binding part according to any one of claims 1-9, the multispecific antibody or the binding fragment thereof according to claim 10, the chimeric antigen receptor according to claim 11, the nucleic acid according to claim 12, or the host cell according to claim 13, for use in the manufacture of a medicament for treating a disease associated with the expression of Claudin 18.2.

18. The use according to claim 17, **characterized in that** the disease associated with the expression of Claudin 18.2 is gastric cancer, pancreatic cancer, oesophageal cancer, breast cancer, colon cancer, liver cancer, ovarian cancer, or lung cancer.

ELISA EC50 assay results of fully human antibodies and human Claudin 18.2 proteins

*FIG. 1*

FACS EC50 assay of fully human antibodies and HEK293/human Claudin 18.2 cells

*FIG. 2*

FACS EC50 assay of fully human antibodies and
HEK293/murine Claudin 18.2 cells

| | |
|---|---|
| —●— | AHP29153 |
| —■— | AHP31548 |
| —▲— | AHP32913 |
| —☐— | AHP32919 |
| —△— | AHP32982 |
| —▽— | AHP33007 |
| —◆— | AHP33029 |
| —●— | AHP30826 |
| —★— | IMAB362 positive reference antibody |
| —+— | Negative isotype control |

*FIG. 3*

FACS EC50 assay of fully human antibodies and
HEK293/human Claudin 18.1 cells

| | |
|---|---|
| —●— | AHP29153 |
| —■— | AHP31548 |
| —▲— | AHP32913 |
| —☐— | AHP32919 |
| —△— | AHP32982 |
| —▽— | AHP33007 |
| —◆— | AHP33029 |
| —●— | AHP30826 |
| —★— | IMAB362 positive reference antibody |
| —+— | Negative isotype control |

*FIG. 4*

FIG. 5

Epitope assay of fully human antibodies based on IMAB362

FIG. 6

In vitro CDC assay results of fully human antibodies

FIG. 7

*In vitro* ADCC assay of fully human antibodies

*FIG. 8*

# INTERNATIONAL SEARCH REPORT

| | International application No. |
|---|---|
| | **PCT/CN2024/121620** |

| | |
|---|---|
| **A.** | **CLASSIFICATION OF SUBJECT MATTER** |

C07K16/28(2006.01)i; C07K16/46(2006.01)i; C07K19/00(2006.01)i; C12N15/13(2006.01)i; C12N15/62(2006.01)i; A61K39/00(2006.01)i; A61P35/00(2006.01)i

According to International Patent Classification (IPC) or to both national classification and IPC

| | |
|---|---|
| **B.** | **FIELDS SEARCHED** |

Minimum documentation searched (classification system followed by classification symbols)

IPC: C07K, C12N, A61K, A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pubmed, STN on web, NCBI, 中文专利序列检索系统, National Sequence Database of Chinese Patent, genbank, EMBL-EBI, 南京蓬勃生物科技有限公司, 李应宇, Claudin 18.2, 紧密连接蛋白18.2, CLDN 18.2, SEQ ID NOs: 1-47

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| PX | CN 117304323 A (NANJING PROBIO BIOTECHNOLOGY CO., LTD.) 29 December 2023 (2023-12-29)<br>entire document | 1-18 |
| Y | WO 2023070201 A1 (FUSION PHARMACEUTICALS INC.) 04 May 2023 (2023-05-04)<br>claims 1-30, and description, embodiments 1-3 | 1, 3-4, 6-18 |
| Y | WO 2022111425 A1 (SANYOU BIOPHARMACEUTICALS CO., LTD.) 02 June 2022 (2022-06-02)<br>claims 1-20, and description, embodiments 1-3 | 1, 3-4, 6-18 |
| Y | WO 2020147451 A1 (ZHEJIANG DOER BIOLOGICS CORPORATION) 23 July 2020 (2020-07-23)<br>claims 1-20 | 1, 3-4, 6-18 |
| Y | WO 2023070202 A1 (FUSION PHARMACEUTICALS INC.) 04 May 2023 (2023-05-04)<br>claims 18-38, and description, embodiment 1 | 1, 3-4, 6-18 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **28 November 2024** | **02 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/121620** |

### C.    DOCUMENTS CONSIDERED TO BE RELEVANT

| Category* | Citation of document, with indication, where appropriate, of the relevant passages | Relevant to claim No. |
|---|---|---|
| Y | CN 115947851 A (CHINA RESOURCES PHARMACEUTICAL CO., LTD.) 11 April 2023 (2023-04-11)<br>        claims 1-20 | 1, 3-4, 6-18 |
| A | WO 2021239026 A1 (HANGZHOU BANGSHUN PHARMACEUTICAL CO., LTD.) 02 December 2021 (2021-12-02)<br>        entire document | 1-18 |

Form PCT/ISA/210 (second sheet) (July 2022)

**INTERNATIONAL SEARCH REPORT**

International application No.

**PCT/CN2024/121620**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2024/121620** |

**Box No. II   Observations where certain claims were found unsearchable (Continuation of item 2 of first sheet)**

This international search report has not been established in respect of certain claims under Article 17(2)(a) for the following reasons:

1. ☑  Claims Nos.: **15-16**
   because they relate to subject matter not required to be searched by this Authority, namely:

   Claims 15-16 relate to a method for treating a disease as defined in PCT Rule 39.1(iv), but a search opinion is still provided on the basis of the pharmaceutical use thereof.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2024/121620**

| Patent document cited in search report | | | Publication date (day/month/year) | Patent family member(s) | | | Publication date (day/month/year) |
|---|---|---|---|---|---|---|---|
| CN | 117304323 | A | 29 December 2023 | None | | | |
| WO | 2023070201 | A1 | 04 May 2023 | AR | 127445 | A1 | 24 January 2024 |
| | | | | EP | 4423136 | A1 | 04 September 2024 |
| WO | 2022111425 | A1 | 02 June 2022 | AU | 2021387558 | A1 | 29 June 2023 |
| | | | | US | 2024092891 | A1 | 21 March 2024 |
| | | | | JP | 2023551027 | A | 06 December 2023 |
| | | | | CA | 3200052 | A1 | 02 June 2022 |
| | | | | EP | 4253411 | A1 | 04 October 2023 |
| | | | | EP | 4253411 | A4 | 30 October 2024 |
| WO | 2020147451 | A1 | 23 July 2020 | EP | 3913001 | A1 | 24 November 2021 |
| | | | | EP | 3913001 | A9 | 12 January 2022 |
| | | | | EP | 3913001 | A4 | 03 August 2022 |
| | | | | EP | 3913001 | B1 | 11 September 2024 |
| | | | | JP | 2022516809 | A | 02 March 2022 |
| | | | | JP | 7061235 | B2 | 27 April 2022 |
| | | | | AU | 2019422629 | A1 | 02 September 2021 |
| | | | | AU | 2019422629 | B2 | 06 October 2022 |
| | | | | US | 2022259303 | A1 | 18 August 2022 |
| | | | | US | 11560427 | B2 | 24 January 2023 |
| WO | 2023070202 | A1 | 04 May 2023 | EP | 4423137 | A1 | 04 September 2024 |
| | | | | AR | 127444 | A1 | 24 January 2024 |
| CN | 115947851 | A | 11 April 2023 | None | | | |
| WO | 2021239026 | A1 | 02 December 2021 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311281157

### Non-patent literature cited in the description

- **CHEN et al.** *J. Mol Biol*, 1999, vol. 293, 865-881
- Fundamental Immunology Second Edition. Raven Press, 1989, 332-336
- **KABAT et al.** Sequences of Proteins of Immunological Interest. 1991
- **AL-LAZIKANI et al.** *J. Mol. Biol.*, 1997, vol. 273 (4), 927-48